# EUROPÄISCHE PATENTANMELDUNG

(11) **EP 2 545 847 A1**
(43) Veröffentlichungstag der Anmeldung: **16.01.2013**
(21) Anmeldenummer: 12187835.9
(22) Anmeldetag: 15.10.2009
(51) Int. Cl.: A61B 5/00

(54) **Erkennen einer Lungenkrebserkrankung mithilfe eines Hundes**

(30) Priorität: 16.10.2008 AT 16242008
(62) Teilanmeldung aus: 09173182.8
(71) Anmelder: Darwin GmbH, 8740 Zeltweg (AT)
(72) Erfinder: Gleichweit, Wolfgang, 8130 Frohnleiten (AT)
(74) Vertreter: Wirnsberger, Gernot

(57) **Zusammenfassung**

Die Erfindung betrifft ein Verfahren zum Erkennen einer Abweichung in einer Atemluft eines ersten Menschen von einer Normatemluft infolge einer Lungenkrebserkrankung desselben mithilfe eines Hundes, umfassend folgende Schritte:
a) Bereitstellen zumindest eines ersten Behältnisses, in welchem ein Adsorptionsmittel gelagert ist, das mit Atemluft eines gemäß Diagnose an Lungenkrebs erkrankten zweiten Menschen in Kontakt stand;
b) Bereitstellen eines weiteren Behältnisses, in welchem ein Adsorptionsmittel gelagert ist, das mit Atemluft des ersten Menschen in Kontakt stand;
c) Führen des Hundes zum ersten Behältnis oder umgekehrt;
d) Öffnen des ersten Behältnisses, sodass der Hund am offenen Behältnis schnuppern kann und optional Belohnen des Hundes;
e) Führen des Hundes zum weiteren Behältnis oder umgekehrt und Öffnen des Behältnisses, sodass der Hund am offenen Behältnis schnuppern kann;
f) Kennzeichnen des weiteren Behältnisses als von der Normatemluft abweichend, wenn der Hund bellt.

Des Weiteren betrifft die Erfindung ein Verfahren zum Konditionieren eines Hundes auf Erkennen einer Lungenkrebserkrankung eines Menschen, eine Verwendung eines Hundes sowie ein Verfahren zur Gewinnung einer Probe zum Erkennen einer Lungenkrebserkrankung mittels eines Hundes.

## Beschreibung

Die Erfindung betrifft ein Verfahren zum Erkennen einer Abweichung in einer Atemluft eines ersten Menschen von einer Normatemluft infolge einer Lungenkrebserkrankung desselben mithilfe eines Hundes.

Des Weiteren betrifft die Erfindung ein Verfahren zum Konditionieren eines Hundes auf Erkennen einer Lungenkrebserkrankung eines Menschen.

Ferner betrifft die Erfindung eine Verwendung eines Hundes.

Darüber hinaus betrifft die Erfindung ein Verfahren zur Gewinnung einer Probe zum Erkennen einer Lungenkrebserkrankung mittels eines Hundes.

Lungenkrebserkrankungen, die beispielsweise auf regelmäßigen Konsum von Zigaretten zurückzuführen sind, sind heute eine häufige Todesursache von Menschen. Eine Lungenkrebserkrankung kann zwar unter Einsatz geeigneter Hilfsmittel bzw. Apparaturen bereits in einem frühen Stadium diagnostiziert werden, allerdings kommt in der Regel aus Kostengründen vor einer eingehenden Untersuchung eine bloße Durchleuchtung einer Lunge eines Menschen bzw. Patienten mit Röntgenstrahlen zum Einsatz. Dabei ist allerdings nachteilig, dass mit diesem Verfahren lediglich bereits relativ große Geschwüre, die auf eine Lungenkrebserkrankung zurückzuführen sind, detektiert werden können. Kleinere Geschwüre in einem früheren Stadium sind zwar wie erwähnt mit aufwendigeren Verfahren bzw. Apparaturen nachweisbar, allerdings werden solche Verfahren bzw. Apparaturen aufgrund hoher Kosten bei einer Erstuntersuchung in der Regel nicht eingesetzt.

Hier setzt die Erfindung an. Aufgabe der Erfindung ist es, ein Verfahren anzugeben, mit welchem auf einfache Weise mit hoher Wahrscheinlichkeit eine mögliche Lungenkrebserkrankung erkannt werden kann, sodass entschieden werden kann, ob ein Patient einer genaueren Untersuchung unter Zuhilfenahme aufwendigerer Verfahren bzw. Apparaturen zugeführt werden soll.

Diese Aufgabe wird gelöst durch ein Verfahren zum Erkennen einer Abweichung in einer Atemluft eines ersten Menschen von einer Normatemluft infolge einer Lungenkrebserkrankung desselben mithilfe eines Hundes, umfassend folgende Schritte:
a) Bereitstellen zumindest eines ersten Behältnisses, in welchem ein Adsorptionsmittel gelagert ist, das mit Atemluft eines gemäß Diagnose an Lungenkrebs erkrankten zweiten Menschen in Kontakt stand;
b) Bereitstellen eines weiteren Behältnisses, in welchem ein Adsorptionsmittel gelagert ist, das mit Atemluft des ersten Menschen in Kontakt stand;
c) Führen des Hundes zum ersten Behältnis oder umgekehrt;
d) Öffnen des ersten Behältnisses, sodass der Hund am offenen Behältnis schnuppern kann und optional Belohnen des Hundes;
e) Führen des Hundes zum weiteren Behältnis oder umgekehrt und Öffnen des Behältnisses, sodass der Hund am offenen Behältnis schnuppern kann;
f) Kennzeichnen des weiteren Behältnisses als von der Normatemluft abweichend, wenn der Hund bellt.

Die mit der Erfindung erzielten Vorteile sind insbesondere darin zu sehen, dass mithilfe eines Hundes, der naturgemäß einen besonders guten Geruchssinn hat, auf einfache Weise mit einer gewissen Wahrscheinlichkeit festgestellt werden kann, ob es zweckmäßig ist, einen Patienten einer aufwendigeren Untersuchung zuzuführen, sodass aufwendige Untersuchungs- und Diagnostizierverfahren kosteneffizient angewendet werden können. Als Normatemluft ist eine Atemluft eines Menschen zu verstehen, bei dem eine Lungenkrebserkrankung auch mit aufwendigsten Verfahren nicht nachweisbar ist.

Bevorzugt ist es, dass mehrere weitere Behältnisse, die ein Adsorptionsmittel enthalten, das jeweils mit Atemluft eines ersten Menschen in Kontakt stand, in einer geraden Linie angeordnet werden. In diesem Fall ist es möglich, dass der eingesetzte Hund sogleich mehrere Behältnisse überprüft. Dabei ist es insbesondere zweckmäßig, dass mehrere erste Behältnisse vor den weiteren Behältnissen angeordnet werden, damit der Hund die aufzuspürenden Gerüche erkennt.

Versuche haben gezeigt, dass in mehr als 70 % eine nachfolgende nähere Untersuchung sowie Diagnose mit einer Bewertung durch den Hund übereinstimmt, sodass, auch wenn das Ergebnis in Einzelfällen nicht zutreffend sein mag, insgesamt doch mit hoher Wahrscheinlichkeit bei Anschlagen des Hundes eine nachfolgende genauere Untersuchung und Diagnose zu einem positiven Befund führt. Dies wiederum resultiert darin, dass gegebenenfalls eine Behandlung einer möglichen Lungenkrebserkrankung rechtzeitig in Angriff genommen werden kann, sodass die Behandlung auch zu einem erfolgreichen Abschluss einer Therapie im Sinne einer Heilung führt.

Zweckmäßig kann es sein, dass mehrere weitere Behältnisse, die ein Adsorptionsmittel enthalten, das jeweils mit Atemluft eines ersten Menschen in Kontakt stand, in einer geraden Linie angeordnet werden. Es ist dann möglich, durch den Hund mehrere Behältnisse bzw. Proben bewerten zu lassen. Im Zusammenhang damit ist es besonders bevorzugt, dass mehrere erste Behältnisse vor den weiteren Behältnissen angeordnet werden, sodass der Hund die festzustellenden Gerüche leicht erkennt.

Zweckmäßig ist es, damit der Hund besonders rasch anschlägt, dass dieser vor Beschnuppern einzelner Proben darauf konditioniert wird, eine mögliche Lungenkrebserkrankung eines Menschen zu erkennen, welches Ziel durch ein Verfahren gemäß Anspruch 4 erreicht wird.

Die mit einem solchen Verfahren erzielten Vorteile sind insbesondere darin zu sehen, dass ein Hund mit einfachen Mitteln so trainiert wird, dass dieser auf einfache Weise mit einer gewissen Wahrscheinlichkeit durch sein Verhalten eine richtige Aussage bezüglich einer möglichen Lungenkrebserkrankung eines Menschen anzeigen kann, und zwar anhand einer mit geringem Aufwand herzustellenden Probe einer Atemluft, die mit einem Adsorptionsmittel in Kontakt gebracht wurde.

Von Vorteil ist es, dass die Behältnisse in einer geraden Linie angeordnet werden, damit der Hund einzelne Behältnisse nicht voneinander unterscheiden kann.

Vorgesehen kann auch sein, dass die ersten und/oder weiteren Behältnisse als solche für den Hund nicht erkennbar gekennzeichnet werden, um allfällige Fehler beim Konditionieren des Hundes möglichst auszuschließen.

Entsprechend den vorstehend dargestellten Vorteilen der Erfindung ist es ein weiteres Ziel, eine Verwendung eines Hundes anzugeben, welches Ziel durch eine Verwendung gemäß Anspruch 7 erreicht wird.

Eine Probe einer Atemluft kann bereitgestellt werden, indem in einem mechanisch verschließbaren bzw. mit zumindest einem mechanischen Verschluss ausgestatteten Röhrchen zumindest ein Adsorptionsmittel gelagert wird, das mit Atemluft eines Menschen in Kontakt gebracht wird, sodass eine Probenherstellung für Prüfung mittels des Hundes einfach ist. Bevorzugt ist ein Adsorptionsmittel enthaltend oder bestehend aus Aktivkohle und/oder Silicagel vorgesehen.

Ein weiteres Ziel der Erfindung besteht darin, auf möglichst einfache Weise eine Probe zum Erkennen einer Lungenkrebserkrankung mittels eines Hundes bereitzustellen, welches Ziel durch ein Verfahren gemäß Anspruch 11 erreicht wird. Bevorzugte Varianten eines erfindungsgemäßen Verfahrens sind Gegenstand der Ansprüche 12 bis 14.

Die mit einem erfindungsgemäßen Verfahren zur Gewinnung einer Probe zum Erkennen einer Lungenkrebserkrankung mittels eines Hundes erreichten Vorteile sind insbesondere darin zu sehen, dass auf einfache und kostengünstige Weise mit hoher Wahrscheinlichkeit eine ein richtiges Ergebnis bringende Probe gewonnen werden kann, aufgrund welcher, nach Bewertung durch einen Hund, ein potenziell an Lungenkrebs erkrankter Mensch gegebenenfalls einer näheren Untersuchung und Diagnose zugeführt werden kann.

Im Folgenden ist die Erfindung anhand eines Ausführungsbeispiels noch näher erläutert.

Von an Lungenkrebs erkrankten lebenden Menschen wurde jeweils eine Atemluft in einem Plastiksack gesammelt, wobei ein Volumen des Plastiksacks jeweils vier Liter betrug. Grundsätzlich können Säcke mit einem Volumen von zwei bis zehn Litern eingesetzt werden. Die in den Plastiksäcken gesammelte Atemluft wurde anschließend jeweils durch ein beidseitig offenes Röhrchen gepresst, welches mit Aktivkohle und Silicagel gefüllt war. Die Röhrchen wurden beidseitig verschlossen und bei einer Temperatur von etwa 4 °C oder weniger gelagert.

In einem separaten Schritt wurden gleich aussehende Röhrchen bereitgestellt, die ebenfalls mit Aktivkohle und Silicagel gefüllt wurden, jedoch mit Atemluft von gemäß Diagnose nach eingehender Untersuchung nicht an Lungenkrebs erkrankten Menschen beaufschlagt wurden.

Die mit Atemluft von an Lungenkrebs erkrankten Menschen beaufschlagten Röhrchen wie auch die mit Atemluft von nicht an Lungenkrebs erkrankten Menschen beaufschlagten Röhrchen bzw. Blindproben wurden anschließend verwendet, um einen Hund so lange zu trainieren bzw. konditionieren, bis dieser bei den Röhrchen, die mit Atemluft von an Lungenkrebs erkrankten Menschen beaufschlagt waren, anschlug bzw. bellte.

Nachdem der Hund konditioniert war, wurden von verschiedenen Testpersonen Atemluftproben in der zuvor beschriebenen Weise entnommen und entsprechende Röhrchen in einer Linie angeordnet. Der konditionierte Hund wurde jeweils zu einem Röhrchen geführt, welches dann geöffnet wurde, sodass der Hund daran schnuppern konnte. Jene Röhrchen, bei welchen der Hund anschlug bzw. bellte, wurden gekennzeichnet. Jene Testpersonen, bei deren Röhrchen der Hund anschlug bzw. bellte, wurden anschließend genau auf Lungenkrebs untersucht, wobei sich in mehr als 70 % der Fälle zeigte, dass Lungenkrebs im Frühstadium diagnostiziert werden konnte.

Ähnliche Versuchsergebnisse wurden auch erhalten, wenn der Hund nicht konditioniert war, aber vor den zu testenden Röhrchen etwa drei Röhrchen angeordnet waren, welche mit Atemluft eines an Lungenkrebs erkrankten Menschen beaufschlagt waren und dem Hund vorerst zum Beschnuppern präsentiert wurden. Der Hund schlug dann ebenfalls bei Röhrchen an, die mit Atemluft von mit überwiegender Wahrscheinlichkeit an Lungenkrebs erkrankten Menschen beaufschlagt waren. Es wird vermutet, dass die mit einer Lungenkrebserkrankung verbundene Entartung menschlichen Gewebes zu einer geringfügigen Änderung der Atemluft führt, was vom Hund aufgrund dessen Geruchssinns erkannt werden kann.

## Patentansprüche

1. Verfahren zum Erkennen einer Abweichung in einer Atemluft eines ersten Menschen von einer Normatemluft infolge einer Lungenkrebserkrankung desselben mithilfe eines Hundes, umfassend folgende Schritte:
a) Bereitstellen zumindest eines ersten Behältnisses, in welchem ein Adsorptionsmittel gelagert ist, das mit Atemluft eines gemäß Diagnose an Lungenkrebs erkrankten zweiten Menschen in Kontakt stand;
b) Bereitstellen eines weiteren Behältnisses, in welchem ein Adsorptionsmittel gelagert ist, das mit Atemluft des ersten Menschen in Kontakt stand;
c) Führen des Hundes zum ersten Behältnis oder umgekehrt;
d) Öffnen des ersten Behältnisses, sodass der Hund am offenen Behältnis schnuppern kann und optional Belohnen des Hundes;
e) Führen des Hundes zum weiteren Behältnis oder umgekehrt und Öffnen des Behältnisses, sodass der Hund am offenen Behältnis schnuppern kann;
f) Kennzeichnen des weiteren Behältnisses als von der Normatemluft abweichend, wenn der Hund bellt.

2. Verfahren nach Anspruch 1, wobei mehrere weitere Behältnisse, die ein Adsorptionsmittel enthalten, das jeweils mit Atemluft eines ersten Menschen in Kontakt stand, in einer geraden Linie angeordnet werden.

3. Verfahren nach Anspruch 2, wobei mehrere erste Behältnisse vor den weiteren Behältnissen angeordnet werden.

4. Verfahren zum Konditionieren eines Hundes auf Erkennen einer Lungenkrebserkrankung eines Menschen, umfassend folgende Schritte:
a) Bereitstellen einer Vielzahl von verschlossenen ersten Behältnissen, in welchen jeweils ein Adsorptionsmittel gelagert ist, das mit Atemluft eines gemäß Diagnose an Lungenkrebs erkrankten Menschen in Kontakt stand;
b) Bereitstellen einer Vielzahl von verschlossenen weiteren Behältnissen, in welchen jeweils ein Adsorptionsmittel gelagert ist, das mit Atemluft eines an Lungenkrebs erkrankten Menschen nicht in Kontakt stand, wobei die weiteren Behältnisse die gleiche Form wie die ersten Behältnisse aufweisen;
c) Anordnen der ersten und weiteren Behältnisse in einer zufälligen Abfolge;
d) Führen des Hundes zu einem Behältnis oder umgekehrt;
e) Öffnen des Behältnisses, sodass der Hund am offenen Behältnis schnuppern kann;
f) Belohnen des Hundes, wenn der Hund bellt und es sich um ein erstes Behältnis handelt;
g) optional Verschließen des Behältnisses;
h) Wiederholen der Schritte d) bis g) für die übrigen Behältnisse.

5. Verfahren nach Anspruch 4, wobei die Behältnisse in einer geraden Linie angeordnet werden.

6. Verfahren nach Anspruch 4 oder 5, wobei die ersten und/oder weiteren Behältnisse als solche für den Hund nicht erkennbar gekennzeichnet werden.

7. Verwendung eines Hundes zum Erkennen einer Abweichung in einer Atemluft eines Menschen von einer Normatemluft infolge einer Lungenkrebserkrankung desselben anhand von Proben eines Adsorptionsmittels, das mit der Atemluft des Menschen in Kontakt gebracht wurde.

8. Verwendung von verschließbaren Röhrchen, in welchen ein Adsorptionsmittel gelagert ist, zur Kontaktierung mit Atemluft eines Menschen und nachfolgende Prüfung auf eine Lungenkrebserkrankung mittels eines Hundes.

9. Verwendung nach Anspruch 8, wobei das Adsorptionsmittel Aktivkohle und/oder Silicagel umfasst.

10. Verwendung nach Anspruch 9, wobei das Adsorptionsmittel aus Aktivkohle und/oder Silicagel besteht.

11. Verfahren zur Gewinnung einer Probe zum Erkennen einer Lungenkrebserkrankung mittels eines Hundes, umfassend folgende Schritte:
a) Sammeln einer Atemluft eines Menschen;
b) In-Kontakt-Bringen der gesammelten Atemluft mit einem Adsorptionsmittel;
c) Aufbewahren des Adsorptionsmittels, vorzugsweise in einem verschließbaren Behältnis wie einem Röhrchen.

12. Verfahren nach Anspruch 11, wobei die Atemluft in einem aufblasbaren Sack gesammelt wird.

13. Verfahren nach Anspruch 12, wobei die im Sack gesammelte Atemluft durch ein in einem Behältnis befindliches Adsorptionsmittel gepresst wird.

14. Verfahren nach Anspruch 13, wobei das Adsorptionsmittel Aktivkohle und/oder Silicagel enthält oder aus diesen besteht.
